# EUROPÄISCHE PATENTSCHRIFT

(11) **EP 2 066 285 B1**
(45) Veröffentlichungstag und Bekanntmachung des Hinweises auf die Patenterteilung: **13.08.2014**
(21) Anmeldenummer: 07765595.9
(22) Anmeldetag: 25.06.2007
(51) Int. Cl.: A61K 8/04, A61K 8/22, A61Q 19/10

(54) **NACHSCHÄUMENDES REINIGUNGSPRODUKT MIT MOLEKULAREM SAUERSTOFF**
POST-FOAMING CLEANSER PRODUCT WITH MOLECULAR OXYGEN
PRODUIT NETTOYANT POST-MOUSSANT AVEC OXYGÈNE MOLÉCULAIRE

(30) Priorität: 19.07.2006 DE 102006033797
(43) Veröffentlichungstag der Anmeldung: 10.06.2009
(73) Patentinhaber: Beiersdorf AG, 20253 Hamburg (DE)
(72) Erfinder: RUPPERT, Stephan, 20259 Hamburg (DE); BLATT, Thomas, 22880 Wedel (DE); MUMMERT, Christoph, 29553 Bienenbüttel (DE); KOLBE, Ludger, 21255 Dohren (DE); AECHTNER, Anja, 68165 Mannheim (DE)
(86) Internationale Anmeldenummer: PCT/EP2007/056314
(87) Internationale Veröffentlichungsnummer: WO 2008/009539

(56) Entgegenhaltungen:
- WO-A-03/022238
- WO-A-2005/027869
- US-A- 4 772 427
- STANZL K ET AL: "THE EFFECTIVENESS OF MOLECULAR OXYGEN IN COSNETIC FORMULATIONS" INTERNATIONAL JOURNAL OF COSMETIC SCIENCE, KLUWER ACADEMIC PUBLISHERS, DORDRECHT, NL, Bd. 18, 1996, Seiten 137-150, XP009042673 ISSN: 0142-5463

## Beschreibung

Die Erfindung betrifft ein nachschäumendes kosmetisches Reinigungsprodukt mit molekularem Sauerstoff, insbesondere Duschgel oder Handseifengele aber auch Badekonzentrate zur Abgabe aus einem Aerosolbehälter und Gesichtsreinigungsgele.

Bekannt sind kosmetische und dermatologische Reinigungsformulierungen, die in Druckgasbehältern abgepackt sind und die nach der Applikation selbständig aufschäumen. Diese Formulierungen bestehen aus einer niederviskosen, tensidhaltigen Reinigungslösung, die durch Druckbeaufschlagung mit leicht flüchtigen Gasen in ein Reinigungsgel überführt wird (DE-OS 38 39 349).

Derartige nachschäumende kosmetische Gele werden bei Anwendung zunächst mit Hilfe eines Ausbringmittels gelförmig aus dem Aerosolbehälter auf die Haut aufgebracht und entwickeln erst dort nach kurzer Verzögerung unter dem Einfluss des enthaltenen Nachschäummittels den eigentlichen Schaum. Der Vorteil dieser Zusammensetzungen gegenüber den bekannten fertigen kosmetischen Schäumen, die bereits geschäumt aus dem Aerosolbehälter auf die Haut aufgebracht werden, liegt in einer besseren Benetzung der Haut. Ein entscheidender Nachteil dieser Produkte ist, dass der eigentliche Nachschäumprozess erst einsetzt, wenn der Verbraucher das Produkt mit den Händen aufschäumt, denn erst dann nimmt das Produkt deutlich an Volumen zu. Durch den zeitverzögerten Nachschäumvorgang ist eine adequate Dosierung des Reinigungsprodukts für den Verbraucher praktisch unmöglich und er entnimmt mal zu viel, mal zu wenig Produkt.

Nachschäumende kosmetische Gele sind im Prinzip bekannt. Die US-PS 3,541,581 nennt als essentielle Bestandteile einer solchen Zusammensetzung Wasser, Seife (d. h. wasserlösliche Salze höherer Fettsäuren), Gelstrukturbildner und Nachschäummittel.

Zweckmäßig, jedoch nicht unbedingt erforderlich ist darüber hinaus der Zusatz von kosmetischen Wirk- und Hilfsstoffen. Es ist auch vorgeschlagen worden (US-PS 4,405, 489), auf einen Gelstrukturbildner zu verzichten, jedoch ist in diesem Falle ein spezieller und aufwendiger Prozess für die Herstellung und Abfüllung derartiger Zusammensetzungen erforderlich.

Die beschriebenen nachschäumenden kosmetischen Gel-Zusammensetzungen weisen jedoch insbesondere bei der Herstellung entscheidende Nachteile auf: Als Nachschäummittel werden aliphatische Kohlenwasserstoffe verwendet, vorzugsweise n-Butan, Pentan und Hexan. Diese Verbindungen sind brennbar und bilden mit Luft explosionsfähige Gemische. Daher ist bei der Fertigung ein erhöhter Aufwand für Explosionsschutz und ähnlichen Sicherheitsmassnahmen erforderlich.

Aufgabe der vorliegenden Erfindung war es daher, eine den Anforderungen der Praxis entsprechende nachschäumende kosmetische Gel-Zusammensetzung, zu schaffen, die als Nachschäummittel eine im Luftgemisch nicht explosionsfähige Verbindung enthält und damit eine problemlosere und zugleich auch kostengünstigere Herstellung ermöglicht.

Aufgabe der Erfindung war es weiterhin, auf der Basis der erfindungsgemäßen nachschäumenden kosmetischen Gel-Zusammensetzung weitere nachschäumende kosmetische Produkte zur Verfügung zu stellen, beispielsweise ein nachschäumendes Gel zur Hautpflege oder zur Hautreinigung.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise - durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße nachschäumende Zubereitungen - insbesondere dann, wenn die Gasbläschen zu klein sind, um unter einem Lichtmikroskop erkannt zu werden- auch an der starken Volumenzunahme des Systems erkennbar.

Die Verwendung von Sauerstoff in kosmetischen oder dermatologischen Zubereitungen zur Prophylaxe vor und Behandlung von Hautalterungserscheinungen, wie beispielsweise Falten und Fältchen, Haut- und Gewebeerschlaffung, Störungen in der Hautregeneration, Durchblutungsstörungen der Haut, Altersflecken und dergleichen wurde kürzlich beschrieben (WO 05/27869). Die dort erfundenen Zubereitungen stellen aber allesamt Emulsionen dar, die zu Reinigungszwecken ungeeignet sind.

Die WO02/05754 beschreibt extern applizierbare Zubereitungen, die einen Sauerstoffträger enthalten, der in einer lipoiden Emulsion molekulardispers eingearbeitet ist, sowie deren Verwendung zur externen Behandlung/Prävention von Sauerstoffmangelzuständen der Haut. Sauerstoffträger sind aber teuer und die verwendeten Emulsionen sind zu Reinigungszwecken nicht geeignet.

Überraschend wurde gefunden, dass eine nachschäumende kosmetische Reinigungszubereitung auf wässriger Basis geeignet zur Abgabe aus einem Aerosolbehälter, enthaltend in einer üblichen wässrigen Grundzubereitung a) waschaktive Tenside mit gesättigten Resten, wobei anionische Tenside (ganz besonders bevorzugt LES, MES, Natrium Cocoyl Glutamate, Natrium Methyl Cocoyltaurate, Dinatrium PEG-5 Laurylcitrat Sulfosuccinat), nicht-ionische Tenside (ganz besonders bevorzugt APGs, PEG-7 Glyceryl Cocoate) und amphotere (ganz besonders bevorzugt Cocamidopropyl Betaine) Tenside oder anionische und amphotere Tenside und b) 2,5 bis 25 Vol.-%, bevorzugt 5 bis 20 Vol.-%, besonders bevorzugt 7,5 bis 15 Vol.-% reinen Sauerstoff, den Nachteilen des Standes der Technik abhilft. Solche nachschäumenden Reinigungsprodukte, die komprimierten, molekularen Sauerstoff enthalten, schäumen unmittelbar nach der Entnahme auf ihr endgültiges Volumen auf und erleichtern somit die Dosierung für den Verbraucher erheblich. Außerdem wurde überraschend gefunden, dass der in der Reinigungsformulierung enthaltene molekulare Sauerstoff während der Applikation in die Haut penetriert und dort eine besondere Wirkung hervorruft. Überraschenderweise wird durch den sehr feinen Schaum eine sehr gleichmäßige und dünne Verteilung des Produktes auf der Haut erreicht. Dies führt zu einem für ein wässriges Reinigungsprodukt ungewöhnlichen außerordentlich angenehmen glatten und samtigen Hautgefühl während und nach der Anwendung des Produktes. Die erfindungsgemäßen Zubereitungen stellen somit in jeglicher Hinsicht überaus befriedigende Präparate dar. Die Erfindung umfasst auch eine aufgeschäumte Zubereitung erhältlich durch Expandieren einer oben beschriebenen Zubereitung.

Bevorzugt ist es, wenn zusätzlich ein Gelbildner eingesetzt wird.

Bevorzugt ist es, wenn der Gelbildner quervernetzt ist und besonders bevorzugt gewählt wird aus der Gruppe Acrylates Copolymer, Carbomer, Acrylates/C10-30 Alkyl Acrylate Crosspolymer.

Bevorzugt ist es, wenn die Zubereitung frei von Stärkederivaten ist.

Bevorzugt ist es, wenn die Zubereitung frei von flüchtigen und/oder nichtflüchtigen Kohlenwasserstoffen ist.

Bevorzugt ist es, wenn die Zubereitung frei von ungesättigten Tensiden ist.

Bevorzugt ist es, wenn die Zubereitung frei von kationischen Tensiden ist.

Bevorzugt ist es, wenn die Zubereitung nach Expansion als Schaum vorliegt.

Darüber hinaus konnte durch Experimente an Saugblasen nachgewiesen werden, dass der Sauerstoff in die Haut penetriert, selbst wenn Wasser hinzutritt, was beim Duschen stets vorkommt. Dazu wurden am volaren Unterarm von Probanden durch Anlegen eines Unterdrucks Saugblasen (∅ 5 mm) erzeugt (siehe: Kuhn M, Wolber R, Kolbe L, Schnorr O, Sies H., Solarsimulated radiation induces secretion of IL-6 and production of isoprostanes in human skin in vivo, Arch Dermatol Res. 2006 Apr;297(10):477-9). Eine Sauerstoffelektrode (Needle Type Fiber-Optic Oxygen Microsensor/ Microx TX3, PreSens GmbH, Regensburg) wurde in die Saugblase eingeführt und unterhalb des Saugblasendaches positioniert. Die Konzentration an Sauerstoff innerhalb der Saugblase wurde mit Hilfe der 02-Elektrode gemessen und der Anstieg (Differenz) zwischen dem Kontrollwert (vor Produktauftragung) und der Produktanwendung (in mg/L) bestimmt.

Das Produkt ist nach Austrag aus dem Packmittel für den Bruchteil einer Sekunde transparent und weist dann Schlieren auf. Der Sauerstoff liegt in Form kleiner Gasperlen vor.

Erfindungsgemäß ist auch ein kosmetisches Reinigungsprodukt umfassend ein Packmittel mit einem innenliegenden verformbaren und im wesentlichen sauerstoffdichten Behälter enthaltend eine Zubereitung nach einem der vorangehenden Patentansprüche und einen äußeren, im wesentlichen starren Behälter, der dem inneren Druck standzuhalten vermag, wobei zwischen innerem und äußerem Behälter 5 bis 300 Vol.-%, bezogen auf das Gesamtvolumen der Zubereitung im inneren Behälter, eines Gases (= Primärtreibmittel), gewählt aus der Gruppe Luft, Stickstoff, Helium, Argon, Lachgas und Kohlendioxid, bevorzugt Luft unter einem zum Austragen der Zubereitung im inneren Behälter geeigneten Druck vorliegt. Dabei liegt die Zubereitung in einem Behältnis vor, welches durch das Primärtreibmittel unter Druck gesetzt wird, so dass die Zubereitung beim Öffnen dieses Behältnisses in Freiheit gesetzt wird.

Überraschend hat sich gezeigt, das sich bei der Begasung des Reinigungsprodukts mit Sauerstoff in einem HANSA-Mischer ein sauerstoffbeladenes Gel bildet und nicht - was zu erwarten gewesen wäre - ein Schaum, der einer Abfüllung nicht zugänglich wäre.

Das äußere Packmittel kann besonders bevorzugt aus Aluminium (mit Schutzlack) sein.

Aufgrund der Pumpbarkeit ist es von Vorteil, wenn die Viskositäten der zu begasenden Reinigungsformulierungen 6000 mPas (gemessen mit dem Haake Viskotester VT-02 bei 20 °C) nicht überschreiten.

Erfindungsgemäße Zubereitungen oder Produkte finden Verwendung zur Verbesserung des Aussehens der Haut und/oder der Hautanhangsgebilde, insbesondere zur Steigerung der Mikrozirkulation der Haut, zur Verbesserung der Hautfarbe und des Teints, zur Verbesserung der Geschmeidigkeit der Haut, zur Erhöhung des Glanzes der Haut, zur Verbesserung der Elastizität von Haut, Haaren und/oder Nägeln, zur Erhöhung bzw. Wiederherstellung der Barriereeigenschaften der Haut, zum Schutz der Haut vor Austrocknung, zum Schutz der Haut vor Umwelteinflüssen, zum Schutz der Haut von Kindern und Jugendlichen, zur Erhöhung der Hautzellregeneration, zur Erfrischung des Hauterneuerungseffekts, zur Verjüngung der Hautzellstruktur.

### Beispiele

Folgende Rohstoffe wurden verwendet:

| | |
|---|---|
| Acrylates/C10-30 Alkyl Acrylate Crosspolymer | Carbopol ETD 2020 (Noveon) |
| Hydroxypropyl Starch Phosphate | Structure XL (National Starch) |
| Styrene/Acrylates Copolymer | Acusol OP 301 (Rohm & Haas) |
| Polyethylen | Inducos 14/1 HN (Induchem) |
| Acrylates Copolymer | Aqua SF-1 (Noveon) |
| Carbomer | PAS 80 (Noveon) |

### Duschgele

| | **1** | **2** | **3** | **4** | **5** |
|---|---|---|---|---|---|
| Natrium Laurylethersulfat | 13,2 | 11 | 9,5 | 11 | 9,5 |
| Cocoamidopropyl Betain | 1,65 | 3,3 | 3,8 | 4 | 5 |
| Natrium Cocoylglutamat | 1,25 | 0,75 | 2,5 | 2,5 | 1 |
| PEG-7 Glyceryl Cocoate | 2,5 | 1,5 | 2 | 2 | 1,5 |
| Acrylat Copolymer | - | - | - | 2,2 | 2,6 |
| PEG-40 hydriertes Rizinusöl | 0,4 | 0,5 | 0,8 | 0,7 | 0,9 |
| PEG-200 hydriertes Glycerylpalmitat | 0,1 | 0,5 | 0,3 | - | - |
| Polyquaternium-10 | - | 0,2 | 0,2 | - | 0,1 |
| Ethylenglykol Distearat | 1,5 | - | - | - | - |
| Styrene/Acrylates Copolymer | - | - | 0,9 | - | 1 |
| Natriumchlorid | - | 0,2 | 0,2 | - | - |
| Natriumbenzoat | 0,4 | 0,4 | 0,4 | - | - |
| Natriumsalicylat | 0,4 | 0,4 | 0,4 | - | - |
| Zitronensäure | q.s. | q.s. | q.s. | - | - |
| Phenoxyethanol | - | - | - | 0,6 | 0,6 |
| Methylparaben | - | - | - | 0,4 | 0,3 |
| Propylparaben | - | - | - | 0,2 | 0,3 |
| Natronlauge | - | - | - | q.s. | q.s. |
| Parfum | q.s. | q.s. | q.s. | q.s. | q.s. |
| Wasser | ad 100 | ad 100 | ad 100 | ad 100 | ad 100 |

| | | | | | |
|---|---|---|---|---|---|
| pH eingestellt auf 4,8 - 7 Zur Herstellung des Schaumes werden 85 Vol.-% der Formulierung mit 15 Vol.-% Sauerstoff aufgeschäumt.^{a} ^{a}Das Aufschäumen kann z. B. erfolgen, in dem in die Zubereitungen Gas eingeblasen wird oder sie in der bestreffenden Gasatmosphäre (heftig) geschlagen, geschüttelt, verspritzt oder gerührt werden. | | | | | |

| | **6** | **7** | **8** | **9*** | **10** | **11** |
|---|---|---|---|---|---|---|
| Natrium Laurylethersulfat | 5,5 | 6 | 7 | 5 | 6 | 5,5 |
| Cocoamidopropyl Betain | 5,5 | 5 | 4 | - | 5 | 5,5 |
| Natrium Cocoylglutamat | - | - | 1 | 1,5 | 1 | - |
| PEG-7 Glyceryl Cocoate | - | 2 | - | - | 2 | 1,5 |
| Acrylat Copolymer | - | - | - | - | 2 | 2,4 |
| Polyquaternium-10 | - | - | 0,2 | - | - | 0,2 |
| Polyquaternium-7 | - | 0,3 | - | - | - | - |
| Ethylenglykol Distearat | 1,2 | - | - | 1 | - | 1,2 |
| Styrene/Acrylates Copolymer | - | 1 | - | - | - | - |
| PEG-200 hydriertes Glycerylpalmitat | 1 | 1 | 0,8 | 0,8 | - | 0,2 |
| PEG-40 hydriertes Rizinusöl | 0,4 | 0,4 | 0,4 | 0,3 | 0,3 | 0,4 |
| Natriumchlorid | 0,3 | 0,1 | 0,3 | 0,3 | - | - |
| Natriumsalicylat | 0,4 | 0,4 | 0,3 | 0,4 | - | - |
| Natriumbenzoat | 0,4 | 0,5 | 0,5 | 0,4 | - | - |
| Zitronensäure | q.s. | q.s. | q.s. | q.s. | - | - |
| Phenoxyethanol | - | - | - | - | 0,7 | 0,6 |
| Methylparaben | - | - | - | - | 0,3 | 0,4 |
| Propylparaben | - | - | - | - | 0,3 | 0,4 |
| Natronlauge | - | - | - | - | q.s. | q.s. |
| Parfum | q.s. | q.s. | q.s. | q.s. | q.s. | q.s. |
| Wasser | ad 100 | ad 100 | ad 100 | ad 100 | ad 100 | ad 100 |

| | | | | | | |
|---|---|---|---|---|---|---|
| pH eingestellt auf 4,8 - 7 Zur Herstellung des Schaumes werden 90 Vol.-% der Formulierung mit 10 Vol.-% Sauerstoff aufgeschäumt.^{a} * Beispiel 9 ist ein nicht erfindungsgemäßes Beispiel. | | | | | | |

### Gesichtsreinigungsgele

| | **17*** | **18*** | **19*** | **20*** | **21*** |
|---|---|---|---|---|---|
| Natrium Laurylethersulfat | 1,5 | 2 | 1 | 2,5 | 1,5 |
| Natrium Methyl Cocoyltaurat | 0,6 | 0,5 | 0,9 | 1 | 0,5 |
| Decyl Glucosid | 0,3 | 0,2 | - | - | 0,5 |
| PEG-7 Glyceryl Cocoate | 0,6 | 0,5 | - | 0,7 | 0,4 |
| Carbomer | 1,2 | 1,2 | 1,2 | 1,2 | 1,2 |
| Glycerin | 2 | 2 | 2 | 2 | 2 |
| PEG-40 Hydriertes Rizinusöl | - | - | - | 0,4 | 0,6 |
| Xanthan Gum | 0,25 | 0,25 | 0,25 | 0,25 | 0,25 |
| Styrene/Acrylates Copolymer | - | - | 1 | 2 | - |
| Parabene | 0,3 | 0,4 | 0,3 | 0,3 | 0,4 |
| Phenoxyethanol | 0,7 | 0,6 | 0,7 | 0,7 | 0,6 |
| Natriumhydroxid | q.s. | q.s. | q.s. | q.s. | q.s. |
| Parfum | q.s. | q.s. | q.s. | q.s. | q.s. |
| Wasser | ad 100 | ad 100 | ad 100 | ad 100 | ad 100 |

| | | | | | |
|---|---|---|---|---|---|
| pH eingestellt auf 6,2 - 7,1 Zur Herstellung des Schaumes werden 75 Vol.-% der Formulierung mit 25 Vol.-% Sauerstoff aufgeschäumt.^{a} * Beispiel 17 bis 21 sind nicht erfindungsgemäße Beispiele. | | | | | |

| | **22** | **23** | **24** | **25** | **26** |
|---|---|---|---|---|---|
| Natrium Myrethsulfat | 1,8 | 2 | 2,5 | 2,2 | 2,5 |
| Decyl Glucosid | 2,2 | 2 | 1,8 | 2 | 2 |
| Cocoamidopropyl Betain | 3,5 | 3,5 | 4 | 3 | 4 |
| Acrylates/C10-30 Alkyl Acrylate Crosspolymer | 0,6 | 0,8 | 0,7 | 0,9 | 1 |
| Hydroxypropyl Starch Phosphate | 0,8 | 0,9 | 1 | - | 0,9 |
| PEG-40 Hydriertes Rizinusöl | 0,5 | 0,5 | - | 0,3 | 0,4 |
| PEG-90 Glyceryl Isostearat + Laureth-2 | 0,2 | 0,2 | 0,1 | 0,15 | - |
| Polyquaternium-10 | - | 0,1 | 0,1 | - | - |
| Styrene/Acrylates Copolymer | 1 | - | 2 | - | - |
| Polyethylen | 2 | - | - | 0,5 | - |
| Phenoxyethanol | 1 | 1 | 0,9 | 0,9 | 1 |
| Methylparaben | 0,4 | 0,3 | 0,4 | 0,4 | 0,3 |
| Propylparaben | 0,4 | 0,2 | 0,4 | 0,4 | 0,3 |
| Natriumhydroxid | q.s. | q.s. | q.s. | q.s. | q.s. |
| Parfum | q.s. | q.s. | q.s. | q.s. | q.s. |
| Wasser | ad 100 | ad 100 | ad 100 | ad 100 | ad 100 |

| | | | | | |
|---|---|---|---|---|---|
| pH eingestellt auf 5,9 - 6,5 Zur Herstellung des Schaumes werden 85 Vol.-% der Formulierung mit 15 Vol.-% Sauerstoff aufgeschäumt.^{a} | | | | | |

### Shampoos:

| | **27** | **28** | **29** | **30** | **31** | **32** |
|---|---|---|---|---|---|---|
| Natrium Laurylethersulfat | 10 | 9 | 8 | 9 | 9 | 9,5 |
| Cocamidopropyl Betain | 3 | 4 | 3 | 4 | 3 | 3 |
| Dinatrium PEG-5 Laurylcitrat Sulfosuccinat | 4 | - | 2 | 3 | - | - |
| Polyquaternium-10 | 0.3 | 0.2 | 0.1 | 0.3 | 0.2 | 0.1 |
| Guar Hydroxypropyl-Trimonium Chlorid | 0.1 | - | 0.1 | 0.2 | - | - |
| PEG-3 Distearat | 1.5 | 3 | 4 | 2 | 1.5 | - |
| PEG-40 hydriertes Rizinusöl | 0.4 | 0.3 | 0.3 | 0.4 | 0.6 | 0,6 |
| Natriumsalicylat | 0.4 | 0.4 | 0.4 | 0.4 | 0.2 | - |
| Natriumbenzoat | 0.4 | 0.4 | 0.4 | 0.4 | 0.4 | 0,45 |
| Natriumchlorid | 1.5 | 1.0 | 1.2 | 1.0 | 2.0 | 1.0 |
| Zitronensäure | q.s. | q.s. | q.s. | q.s. | q.s. | q.s. |
| Parfum | q.s. | q.s. | q.s. | q.s. | q.s. | q.s. |
| Wasser | ad 100 | ad 100 | ad 100 | ad 100 | ad 100 | ad 100 |

| | | | | | | |
|---|---|---|---|---|---|---|
| pH eingestellt auf 4,8 - 5,8 Zur Herstellung des Schaumes werden 80 Vol.-% der Formulierung mit 20 Vol.-% Sauerstoff aufgeschäumt.^{a} | | | | | | |

## Patentansprüche

1. Nachschäumende kosmetische Reinigungszubereitung auf wässriger Basis geeignet zur Abgabe aus einem Aerosolbehälter, enthaltend in einer üblichen wässrigen Grundzubereitung
a) Waschaktive Tenside mit gesättigten Resten, wobei die waschaktiven Tenside anionische Tenside, nicht-ionische Tenside und amphotere Tenside oder anionische und amphotere Tenside sind,
b) 2,5 bis 25 Vol.-%, bevorzugt 5 bis 20 Vol.-%, besonders bevorzugt 7,5 bis 15 Vol.-% reinen Sauerstoff.

2. Aufgeschäumte Zubereitung erhältlich durch Expandieren einer Zubereitung nach Anspruch 1.

3. Zubereitung nach einem der vorangehenden Patentansprüche, **dadurch gekennzeichnet, dass** zusätzlich ein Gelbildner eingesetzt wird.

4. Zubereitung nach einem der vorangehenden Patentansprüche, **dadurch gekennzeichnet, dass** der Gelbildner quervernetzt ist und besonders bevorzugt gewählt wird aus der Gruppe Acrylates Copolymer, Carbomer, Acrylates/C10-30 Alkyl Acrylate Crosspolymer.

5. Zubereitung nach einem der vorangehenden Patentansprüche, **dadurch gekennzeichnet, dass** die Zubereitung nach Expansion als Schaum vorliegt.

6. Kosmetisches Reinigungsprodukt umfassend ein Packmittel mit einem innenliegenden verformbaren und im wesentlichen sauerstoffdichten Behälter enthaltend eine Zubereitung nach einem der vorangehenden Patentansprüche und einen äußeren, im wesentlichen starren Behälter, der dem inneren Druck standzuhalten vermag, wobei zwischen innerem und äußerem Behälter 5 bis 300 Vol.-%, bezogen auf das Gesamtvolumen der Zubereitung im inneren Behälter, eines Gases (= Primärtreibmittel), gewählt aus der Gruppe Luft, Stickstoff, Helium, Argon, Lachgas und Kohlendioxid, bevorzugt Luft unter einem zum Austragen der Zubereitung im inneren Behälter geeigneten Druck vorliegt.

7. Nicht-therapeutische Verwendung von kosmetischen Reinigungszubereitungen oder Produkten nach einem der vorhergehenden Ansprüche zur Verbesserung des Aussehens der Haut und/oder der Hautanhangsgebilde, insbesondere zur Steigerung der Mikrozirkulation der Haut, zur Verbesserung der Hautfarbe und des Teints, zur Verbesserung der Geschmeidigkeit der Haut, zur Erhöhung des Glanzes der Haut, zur Verbesserung der Elastizität von Haut, Haaren und/oder Nägeln, zur Erhöhung bzw. Wiederherstellung der Barriereeigenschaften der Haut, zum Schutz der Haut vor Austrocknung, zum Schutz der Haut vor Umwelteinflüssen, zum Schutz der Haut von Kindern und Jugendlichen.

## Claims

1. Post-foaming cosmetic cleanser preparation on an aqueous basis suitable for dispensing from an aerosol container, comprising in a customary aqueous base preparation
a) washing-active surfactants with saturated radicals, where the washing-active surfactants are anionic surfactants, nonionic surfactants and amphoteric surfactants or anionic and amphoteric surfactants,
b) 2.5 to 25% by volume, preferably 5 to 20% by volume, particularly preferably 7.5 to 15% by volume, of pure oxygen.

2. Foamed preparation obtainable by expanding a preparation according to Claim 1.

3. Preparation according to one of the preceding patent claims, **characterized in that** a gel former is additionally used.

4. Preparation according to one of the preceding patent claims, **characterized in that** the gel former is crosslinked and is particularly preferably selected from the group Acrylates Copolymer, Carbomer, Acrylates/C10-30 Alkyl Acrylate Crosspolymer

5. Preparation according to one of the preceding patent claims, **characterized in that** the preparation is in the form of a foam after expansion.

6. Cosmetic cleansing product comprising a packaging means with an inner deformable and essentially oxygen-tight container containing a preparation according to one of the preceding patent claims and an outer, essentially rigid container which is able to withstand the internal pressure, where, between the inner and outer container, 5 to 300 % by volume, based on the total volume of the preparation in the inner container, of a gas (= primary propellant), selected from the group air, nitrogen, helium, argon, nitrous oxide and carbon dioxide, preferably air, is present between the inner and outer container under a pressure suitable for discharging the preparation in the inner container.

7. Non-therapeutic use of cosmetic cleansing preparations or products according to one of the proceeding claims for improving the appearance of the skin and/or the skin appendages, in particular for increasing the microcirculation of the skin, for improving the colour of the skin and the complexion, for improving the suppleness of the skin, for increasing the shine of the skin, for improving the elasticity of skin, hair and/or nails, for increasing and/or restoring the barrier properties of the skin, for protecting the skin from drying out, for protecting the skin against environmental influences, for protecting the skin of children and adolescents.

## Revendications

1. Composition de nettoyage cosmétique post-moussante à base aqueuse appropriée pour être distribuée à partir d'un récipient à aérosol, contenant, dans une composition de base aqueuse usuelle
a) des agents tensioactifs actifs en lavage présentant des radicaux saturés, les agents tensioactifs actifs en lavage étant des agents tensioactifs anioniques, des agents tensioactifs non ioniques et des agents tensioactifs amphotères ou des agents tensioactifs anioniques et amphotères,
b) 2,5 à 25% en volume, de préférence 5 à 20% en volume, de manière particulièrement préférée 7,5 à 15% en volume d'oxygène pur.

2. Composition moussée pouvant être obtenue par dilatation d'une composition selon la revendication 1.

3. Composition selon l'une quelconque des revendications précédentes, **caractérisée en ce qu'**on utilise en plus un gélifiant.

4. Composition selon l'une quelconque des revendications précédentes, **caractérisée en ce que** le gélifiant est réticulé et est choisi de manière particulièrement préférée dans le groupe formé par un copolymère d'acrylates, un carbomére, un copolymère d'acrylate/acrylate de C₁₀₋₃₀-alkyle.

5. Composition selon l'une quelconque des revendications précédentes, **caractérisée en ce que** la composition se trouve sous forme de mousse après la dilatation.

6. Produit de nettoyage cosmétique comprenant un emballage et un récipient interne déformable et essentiellement étanche à l'oxygène, contenant une composition selon l'une quelconque des revendications précédentes et un récipient externe, essentiellement rigide, qui peut résister à la pression interne, 5 à 300% en volume, par rapport au volume total de la composition dans le récipient interne, d'un gaz (agent propulseur primaire), choisi dans le groupe formé par l'air, l'azote, l'hélium, l'argon, le protoxyde d'azote et le dioxyde de carbone, de préférence de l'air, se trouvant entre le récipient interne et le récipient externe, sous une pression appropriée pour distribuer la composition se trouvant dans le récipient interne.

7. Utilisation non thérapeutique de compositions de nettoyage ou de produits cosmétiques selon l'une quelconque des revendications précédentes pour améliorer l'aspect de la peau et/ou des annexes de la peau, en particulier pour augmenter la microcirculation de la peau, pour améliorer la couleur et le teint de la peau, pour améliorer la souplesse de la peau, pour augmenter la brillance de la peau, pour améliorer l'élasticité de la peau, des cheveux et/ou des ongles, pour augmenter ou réparer les propriétés de barrière de la peau, pour protéger la peau contre un dessèchement, pour protéger la peau contre les influences environnementales, pour protéger la peau des enfants et desjeunes.
